# EUROPEAN PATENT SPECIFICATION

(11) **EP 2 964 115 B1**
(45) Date of publication and mention of the grant of the patent: **30.10.2019**
(21) Application number: 14759560.7
(22) Date of filing: 07.03.2014
(51) Int. Cl.: A61B 17/58, A61B 17/70

(54) **BONE FASTENER**
KNOCHENFIXIERVORRICHTUNG
ELÉMENT DE FIXATION OSSEUSE

(30) Priority: 08.03.2013 US 201313791199
(43) Date of publication of application: 13.01.2016
(73) Proprietor: Warsaw Orthopedic, Inc., Warsaw, IN 46582 (US)
(72) Inventor: REZACH, William Alan, Atoka, TN 38004 (US)
(74) Representative: Viering, Jentschura & Partner mbB Patent- und Rechtsanwälte
(86) International application number: PCT/US2014/021871
(87) International publication number: WO 2014/138614

(56) References cited:
- WO-A1-2012/030712
- US-A1- 2004 267 264
- US-A1- 2007 088 357
- US-A1- 2008 119 858
- US-A1- 2010 312 288
- US-A1- 2011 071 577
- US-A1- 2012 046 701
- US-A1- 2012 109 218
- US-A1- 2012 185 003
- US-A1- 2012 277 805

## Description

The present disclosure generally relates to medical devices for the treatment of spinal disorders, and more particularly to a surgical implant system including a bone fastener that provides stabilization while reducing stress on spinal elements.

### BACKGROUND

Spinal pathologies and disorders such as scoliosis and other curvature abnormalities, kyphosis, degenerative disc disease, disc herniation, osteoporosis, spondylolisthesis, stenosis, tumor, and fracture may result from factors including trauma, disease and degenerative conditions caused by injury and aging. Spinal disorders typically result in symptoms including deformity, pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders includes correction, fusion, fixation, discectomy, laminectomy and implantable prosthetics. As part of these surgical treatments, spinal constructs such as vertebral rods are often used to provide stability to a treated region. Rods redirect stresses away from a damaged or defective region while healing takes place to restore proper alignment and generally support the vertebral members. During surgical treatment, one or more rods and bone fasteners can be delivered to a surgical site. The rods may be attached via the fasteners to the exterior of two or more vertebral members. This disclosure describes an improvement over these prior art technologies. As further spinal constructs for surgical treatments, a spinal implant with a lockable ball joint is known from US 2012/0046701 A1, a multi-axial screw assembly is known from US 2012/0109218 A1 and a polyaxial pedicle screw is known from WO 2012/030712 A1.

### SUMMARY

To this end, the present invention provides a bone fastener according to claim 1. Further embodiments of the present invention are described in the dependent claims..

### BRIEF DESCRIPTION OF THE DRAWINGS

The present disclosure will become more readily apparent from the specific description accompanied by the following drawings, in which:
FIG. 1 is a perspective view of components of one embodiment of a spinal implant system in accordance with the principles of the present disclosure;
FIG. 2 is a perspective view of the components shown in FIG. 1 with parts separated;
FIG. 3 is a perspective view of a component of the spinal implant system shown in FIG. 1;
FIG. 4 is a perspective view of a component of the spinal implant system shown in FIG. 1;
FIG. 5 is a perspective view of components of the spinal implant system shown in FIG. 1;
FIG. 6 is a cross section view of the components taken along lines A-A shown in FIG. 5;
FIG. 7 is a break away, cross section view of components of the spinal implant system shown in FIG. 1;
FIG. 8 is a break away, cross section view of components of the spinal implant system shown in FIG. 1;
FIG. 9 is a break away view of components of the spinal implant system shown in FIG. 1;
FIG. 10 is a break away, cross section view of components taken along lines B-B in FIG. 13;
FIG. 11 is a break away, cross section view of components of the spinal implant system shown in FIG. 1;
FIG. 12 a cross section, end view of components taken along lines C-C in FIG. 13;
FIG. 13 is a break away view of components of the spinal implant system shown in FIG. 1 with parts separated;
FIG. 14 is a break away view of components of the spinal implant system shown in FIG. 1;
FIG. 15 is a break away, cross section view of components taken along lines D-D in FIG. 14;
FIG. 16 is a break away view of components of the spinal implant system shown in FIG. 1;
FIG. 17 is a break away view of components of the spinal implant system shown in FIG. 1;
FIG. 18 is a break away, cross section view of the components taken along lines E-E in FIG. 17;
FIG. 19 is a break away, perspective view of components of one embodiment of a spinal implant system in accordance with the principles of the present disclosure with parts separated;
FIG. 20 is a cross section view of the components shown in FIG. 19;
FIG. 21 is a perspective view of components of the spinal implant system shown in FIG. 19 with parts separated;
FIG. 22 is an end view of one embodiment of components of the spinal implant system shown in FIG. 19;
FIG. 23 is a side view of components of the spinal implant system;
FIG. 24 is an end view of components of the spinal implant system;
FIG. 25 is a plan view of vertebrae showing a trajectory for the spinal implant system;
FIG. 26 is a plan view of components of one embodiment of a spinal implant system in accordance with the principles of the present disclosure disposed with vertebrae;
FIG. 27 is a side view of components of one embodiment of a spinal implant system in accordance with the principles of the present disclosure with parts separated;
FIG. 28 is a perspective view of components of one embodiment of a spinal implant system in accordance with the principles of the present disclosure with parts separated;
FIG. 29 is a break away view of components of the spinal implant system shown in FIG. 28 with parts separated; and
FIG. 30 is a cross section view of the components shown in FIG. 28.

### DETAILED DESCRIPTION

The exemplary embodiments of a surgical system and related methods of use disclosed are discussed in terms of medical devices for the treatment of musculoskeletal disorders and more particularly, in terms of a spinal implant system including a bone fastener, wherein said methods of use do not form part of the invention as such but help explaining aspects of the invention. In one embodiment, the spinal implant system includes a S2-alar-iliac biased angle bone fastener including a threaded screw
configured to penetrate tissue, such as, for example, bone and a head including an inner surface defining an implant cavity. In some embodiments, the head is rotatable relative to the screw such that the implant cavity can rotate at least 180 degrees in the coronal plane. This configuration allows the screw to be fixed within tissue at a desired trajectory and the head rotated relative to the screw to align the implant cavity with an implant, such as for example, a vertebral rod to position the rod within the implant cavity. In some embodiments, the screw allows for 45 degrees of angulation in a biased plane and allows the biased plane to rotate in the coronal plane. In some embodiments, the spinal implant system can be employed with surgical procedures using midline laminectomy trajectories.

In one embodiment, the surgical system includes a bone fastener that includes a crown having a single flat for keying into a base. In one embodiment, the bone fastener includes a ring having a cylindrical shape. In one embodiment, the bone fastener includes a ring having an elliptical shape. In one embodiment, the bone fastener is assembled by maintaining alignment of a slot of the ring with a cutout of the base.

In one embodiment, the bone fastener is assembled by employing a flexible wire insertion technique. In one embodiment, a sub-assembly slides into the head and a straight metallic wire is inserted through a hole of the head and then deforms into a 'c' type of arrangement. In one embodiment, the wire may include a kink to prevent dis-assembly after insertion into the sub-assembly. In one embodiment, a weld may be used to prevent dis-assembly of the sub-assembly. In one embodiment, the wire may include a larger end such that part of the insertion is press fit into the head.

In one embodiment, the bone fastener includes a pedicle screw that can rotate to approximately 45 degrees and the plane in which it articulates can then rotate to meet a caudal angle aspect of a trajectory. In one embodiment, the pedicle screw can rotate 40-45 degrees on the biased plane. In one embodiment, the pedicle screw can rotate at least 20 degrees in a caudal direction. In one embodiment, the S2-alar-iliac trajectory can include a major alar projection, which forms an upper part of an ilio-pectineal line, can be 40-50 degrees lateral to a horizontal plane of a body, can be 20-30 degrees caudal-guided by a posterior surface of a sacrum of a body and/or can be along an anterior inferior iliac spine, which can be found by palpating the top of a greater trochanter.

In one embodiment, the bone fastener includes a threaded base and an elliptical snap ring. In one embodiment, the bone fastener includes a keyed base and an elliptical snap ring. In one embodiment, the bone fastener includes a threaded base and does not include a snap ring.

In one embodiment, the bone fastener is assembled by inserting a crown, a screw and a snap ring into a base to form a sub-assembly. The sub-assembly is inserted into the head and rotated 90 degrees. In one embodiment, the head includes stake head holes to prevent disassembly of the sub-assembly.

In one embodiment, the surgical system includes a bone fastener including a head, a base configured for fixation with the head, a crown configured for fixation with the base, a ring and a screw. The ring is disposed about a proximal portion of the shaft to engage the screw with the base. The base includes a wall having a lateral surface defining a cut-out. The ring is substantially cylindrical and includes a gap. The gap in the ring is aligned with the cut-out in the base when the ring is disposed about the proximal portion of the screw such that a distal end of the screw can rotate between about 0 degrees to about 180 degrees in the coronal plane relative to an axis defined by the head. In some embodiments, the head is made from a cobalt chrome alloy, the base is made from a cobalt chrome material or a titanium alloy, the crown is made from commercially pure titanium, the ring is made from a cobalt chrome alloy and the screw is made from a titanium alloy.

In one embodiment, an inner surface of the base and a wall of the crown each include a single flat. The flat on the crown is aligned with the flat on the base to key the crown into the base such that the crown is prevented from translating axially relative to the base in at least one direction. In one embodiment, the inner surface of the base and the wall of the crown each have a hexagonal configuration engageable with one another to prevent rotation of the crown relative to the base.

In one embodiment, the head includes an inner surface including a threaded portion and a second portion defining an implant cavity configured for disposal of an implant, such as, for example, a vertebral rod. The base includes a threaded outer surface configured to engage the inner surface of the head to fix the head with the base. In some embodiments, engagement of the threads on the outer surface of the base and the threads on the inner surface of the head may be timed such that disposal of the head and the base in a fixed orientation aligns the implant cavity in a selected direction, such as, for example, in a cephalad direction and/or a caudal direction. In some embodiments, the implant cavity may be positioned proximal of the second portion of the inner surface of the head to avoid compromising the stiffness of the head.

In one embodiment, an outer surface of the base includes a groove and the head is deformable. In some embodiments, the head is fixed to the base by engaging an inner surface of the head with an outer surface of the base such that the head is spaced apart from the groove. In some embodiments, the head is deformed at a stake zone such that at least a portion of the head is positioned within the groove. In one embodiment, the head is fixed with the base by inserting an instrument, such as, for example, a press fit pin through an outer surface of the head and into the recess.

In one embodiment, the head includes an inner surface defining a cylindrical undercut extending transverse to an axis defined by the head and the base includes an outer surface defining a cylindrical undercut extending transverse to the axis defined by the head. The inner surface of the head engages the outer surface of the base such that the cylindrical undercuts are aligned. A wire is positioned in the cylindrical undercuts to fix the head with the base. In one embodiment, the wire is positioned in the cylindrical undercuts through an opening in an outer surface of the head. In one embodiment, the wire is a straight metallic wire that is inserted into the opening in the outer surface of the head and deforms to include a C-shape upon insertion into the cylindrical undercuts. In one embodiment, the wire includes a kink to prevent the base from disengaging the head after the wire is inserted into the cylindrical undercuts.

In one embodiment, the base of the bone fastener is made from a titanium alloy and a cobalt chrome alloy such that the base can resist between 7,000 Newtons of force and 8,000 Newtons of force. In one embodiment, the head has a stiffness sufficient to resist displacement at 500 Newtons of force.

In one embodiment, the bone fastener includes a head made from a cobalt chrome alloy, a base made from a titanium alloy, a crown made from commercially pure titanium, a threaded nut made from a titanium alloy and a screw made from a titanium alloy. The screw includes a substantially spherical proximal portion. The crown includes a projection extending from an outer surface of the crown. The crown is positioned on top of the proximal portion of the screw. The base includes a longitudinal slot and an inner surface defining a passageway. The crown is positioned within the carrier such that the extension is positioned in the slot. In one embodiment, the substantially spherical proximal portion of the screw includes a recess defining a drive portion configured for disposal of a tool, such as, for example, a driver. In one embodiment, the bone fastener is configured for insertion through cortical bone within the sacrum.

In some embodiments, the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. In some embodiments, the present disclosure may be employed with other osteal and bone related applications, including those associated with diagnostics and therapeutics. In some embodiments, the disclosed spinal implant system may be alternatively employed in a surgical treatment with a patient in a prone or supine position, and/or employ various surgical approaches to the spine, including anterior, posterior, posterior mid-line, lateral, postero-lateral, and/or antero-lateral approaches, and in other body regions. The present disclosure may also be alternatively employed with procedures for treating the lumbar, cervical, thoracic, sacral and pelvic regions of a spinal column. The spinal implant system of the present disclosure may also be used on animals, bone models and other non-living substrates, such as, for example, in training, testing and demonstration.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment. It is also understood that all spatial references, such as, for example, horizontal, vertical, top, upper, lower, bottom, left and right, are for illustrative purposes only and can be varied within the scope of the disclosure. For example, the references "upper" and "lower" are relative and used only in the context to the other, and are not necessarily "superior" and "inferior".

Further, as used in the specification and including the appended claims, "treating" or "treatment" of a disease or condition refers to performing a procedure that may include administering one or more drugs to a patient (human, normal or otherwise or other mammal), employing implantable devices, and/or employing instruments that treat the disease, such as, for example, microdiscectomy instruments used to remove portions bulging or herniated discs and/or bone spurs, in an effort to alleviate signs or symptoms of the disease or condition. Alleviation can occur prior to signs or symptoms of the disease or condition appearing, as well as after their appearance. Thus, treating or treatment includes preventing or prevention of disease or undesirable condition (e.g., preventing the disease from occurring in a patient, who may be predisposed to the disease but has not yet been diagnosed as having it). In addition, treating or treatment does not require complete alleviation of signs or symptoms, does not require a cure, and specifically includes procedures that have only a marginal effect on the patient. Treatment can include inhibiting the disease, e.g., arresting its development, or relieving the disease, e.g., causing regression of the disease. For example, treatment can include reducing acute or chronic inflammation; alleviating pain and mitigating and inducing re-growth of new ligament, bone and other tissues; as an adjunct in surgery; and/or any repair procedure. Also, as used in the specification and including the appended claims, the term "tissue" includes soft tissue, ligaments, tendons, cartilage and/or bone unless specifically referred to otherwise.

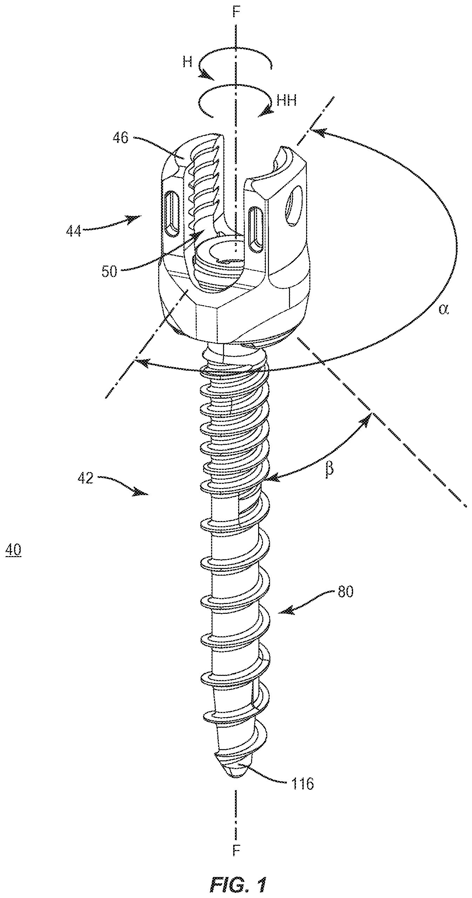

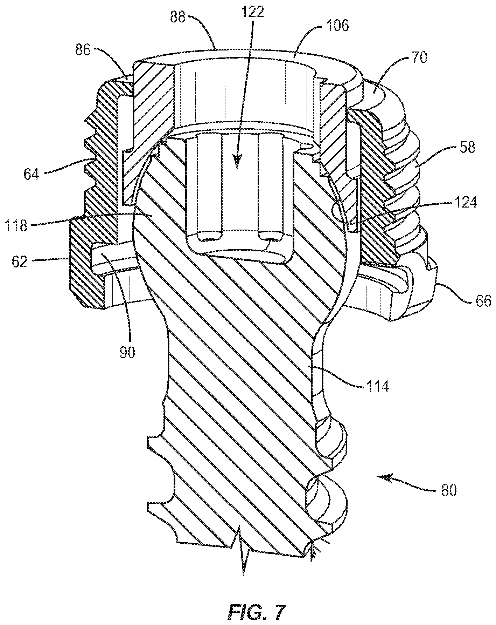

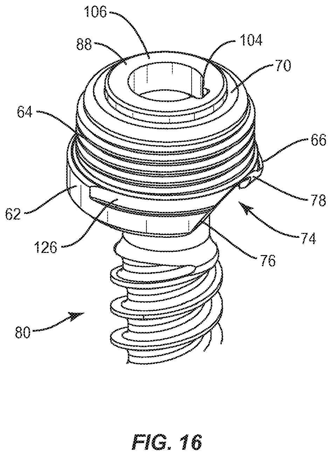

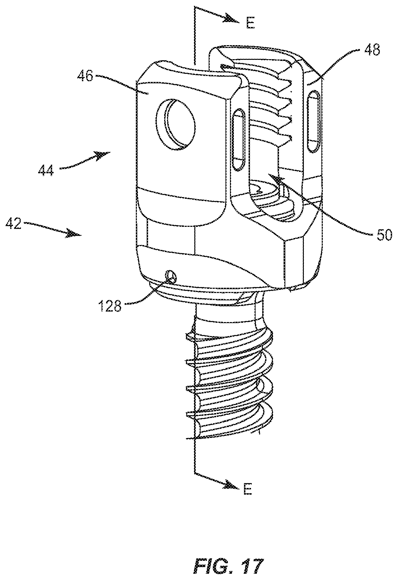

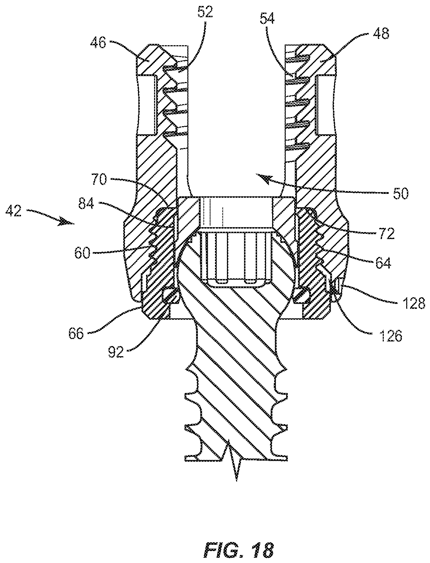

The following discussion includes a description of a surgical system including a bone fastener, related components and methods of employing the surgical system in accordance with the principles of the present disclosure. Alternate embodiments are also disclosed. Reference will now be made in detail to the exemplary embodiments of the present disclosure, which are illustrated in the accompanying figures. Turning to FIGS. 1-18, there are illustrated components of a spinal implant system 40 including a bone fastener 42 in accordance with the principles of the present disclosure.

The components of spinal implant system 40 can be fabricated from biologically acceptable materials suitable for medical applications, including metals, synthetic polymers, ceramics and bone material and/or their composites, depending on the particular application and/or preference of a medical practitioner. For example, the components of spinal implant system 40, individually or collectively, can be fabricated from materials such as stainless steel alloys, commercially pure titanium, titanium alloys, Grade 5 titanium, super-elastic titanium alloys, cobalt-chrome alloys, stainless steel alloys, superelastic metallic alloys (e.g., Nitinol, super elasto-plastic metals, such as GUM METAL® manufactured by Toyota Material Incorporated of Japan), ceramics and composites thereof such as calcium phosphate (e.g., SKELITE™ manufactured by Biologix Inc.), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polyaetide, polyglycolide, polytyrosine carbonate, polycaroplaetohe and their combinations. Various components of spinal implant system 40 may have material composites, including the above materials, to achieve various desired characteristics such as strength, rigidity, elasticity, compliance, biomechanical performance, durability and radiolucency or imaging preference. The components of spinal implant system 40, individually or collectively, may also be fabricated from a heterogeneous material such as a combination of two or more of the above-described materials. The components of spinal implant system 40 may be monolithically formed, integrally connected or include fastening elements and/or instruments, as described herein.

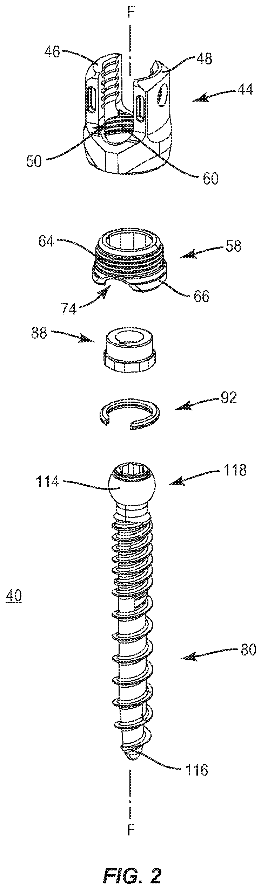

Fastener 42 includes a proximal portion, such as, for example, a head 44 extending along an axis F. Head 44 includes a pair of spaced apart arms 46, 48 that define an implant cavity 50 therebetween configured for disposal of a spinal construct, such as, for example, a spinal rod. Arms 46, 48 each extend parallel to axis F, as shown in FIGS. 1 and 2. In some embodiments, arm 46 and/or arm 48 may be disposed at alternate orientations, relative to axis F, such as, for example, transverse, perpendicular and/or other angular orientations such as acute or obtuse, coaxial and/or may be offset or staggered. Arms 46, 48 each include an arcuate outer surface extending between a pair of side surfaces. At least one of the outer surfaces and the side surfaces of arms 46, 48 have at least one recess or cavity therein configured to receive an insertion tool, compression instrument and/or instruments for inserting and tensioning fastener 42.

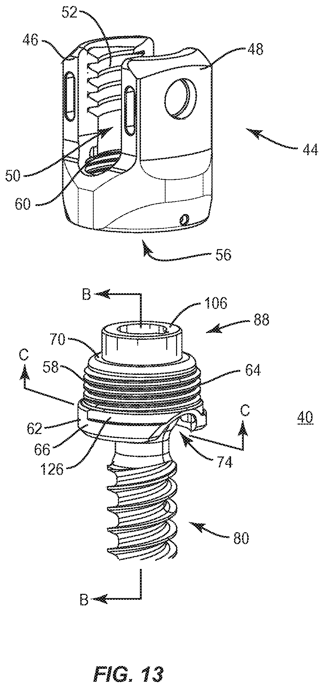

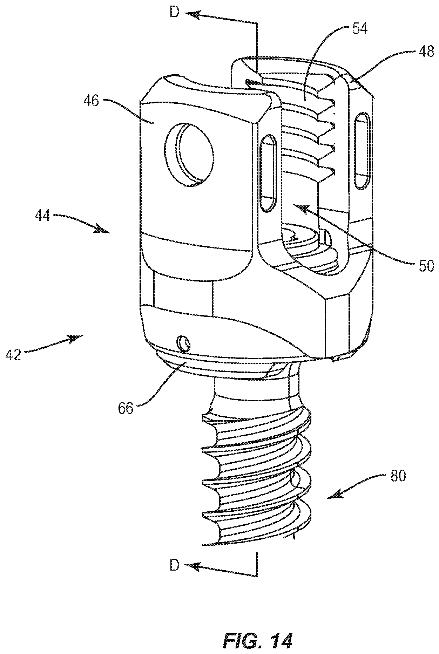

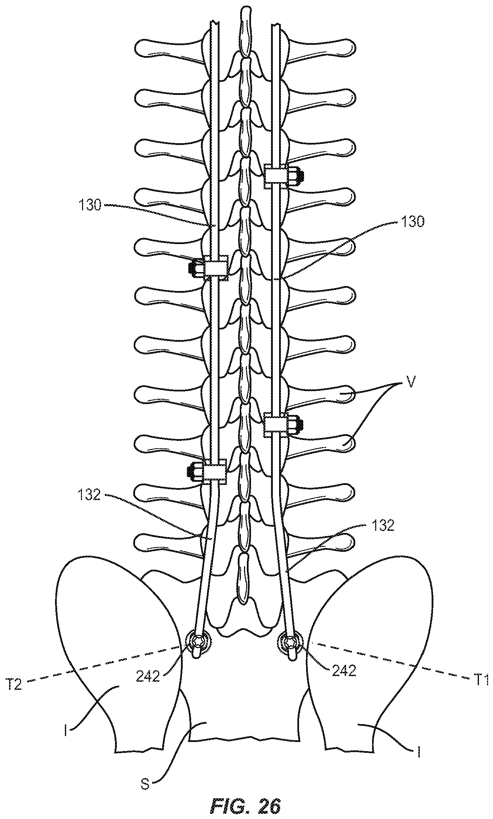

Cavity 50 is substantially U-shaped. In some embodiments, all or only a portion of cavity 50 may have alternate cross section configurations, such as, for example, V-shaped, W-shaped, oval, oblong triangular, square, polygonal, irregular, uniform, non-uniform, offset, staggered, and/or tapered, depending upon the requirements of a particular application. An inner surface of head 44 includes a thread form 52 located adjacent arm 46 and a thread form 54 located adjacent arm 48, as shown in FIGS. 13 and 14. Thread forms 52, 54 are each configured for engagement with a coupling member, such as, for example, a setscrew (FIG. 26), to retain a spinal construct, such as, for example, a spinal rod within cavity 50. In some embodiments, the inner surface of head 44 may be disposed with the coupling member in alternate fixation configurations, such as, for example, friction fit, pressure fit, locking protrusion/recess, locking keyway and/or adhesive. In some embodiments, all or only a portion of the inner surface of head 44 may have alternate surface configurations to enhance engagement with the spinal rod and/or the setscrew such as, for example, rough, arcuate, undulating, mesh, porous, semi-porous, dimpled and/or textured, according to the requirements of a particular application.

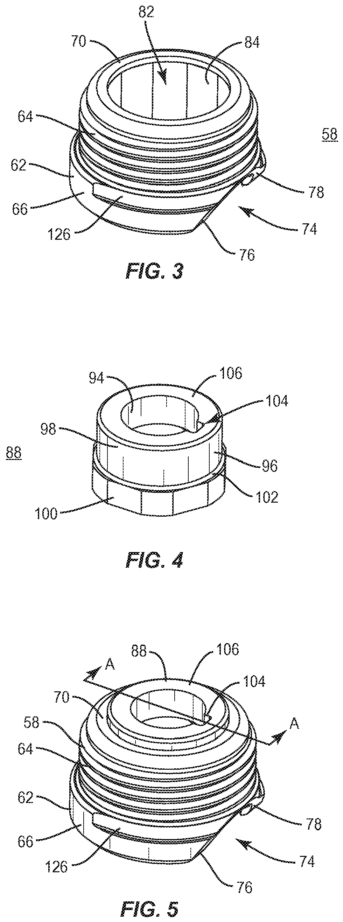

The inner surface of head 44 defines an opening 56, as shown in FIG. 13, configured for disposal of a first member, such as, for example, a base 58, as shown in FIG. 3. The inner surface of head 44 includes a thread form 60, as shown in FIG. 2, located adjacent opening 56 and spaced apart from thread forms 54, 56 configured for engagement with base 58 to fix head 44 with base 58. Thread form 60 has a pitch that is different from the pitch of thread forms 54, 56. In one embodiment, thread form 60 is continuous with thread form 54 and/or thread form 56 and has a pitch that is the same as the pitch of thread forms 54, 56. In some embodiments, head 44 may be disposed with base 58 in alternate fixation configurations, such as, for example, friction fit, pressure fit, locking protrusion/recess, locking keyway and/or adhesive.

Base 58 is configured for fixation with head 44 and includes a wall having a lateral surface 62 including a threaded portion 64. Portion 64 includes a thread form configured for threaded engagement with thread form 60 to fix head 44 with base 58. Thread form 60 and the thread form on portion 64 are timed such that cavity 50 is aligned in a cephalad direction and/or a caudal direction of a body when thread form 60 fully engages the thread form on portion 64. In some embodiments, thread form 60 and the thread form on portion 64 are timed such that cavity 50 is aligned in a lateral direction when thread form 60 fully engages the thread form on portion 64.

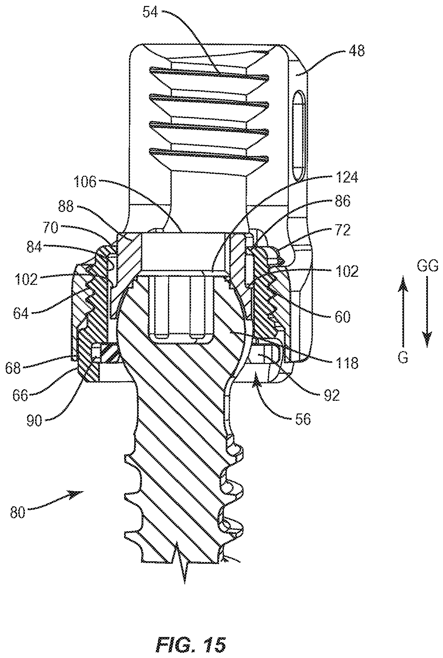

Surface 62 includes an unthreaded portion 66 positioned adjacent portion 64. The thread form on portion 64 has a major diameter that is less than a maximum diameter of portion 66 such that portion 66 engages an unthreaded distal portion 68 of opening 56 that extends parallel to axis F and is spaced apart from thread form 60 when thread form 60 fully engages the thread form on portion 64, as shown in FIG. 15.

When thread form 60 fully engages the thread form on portion 64, a proximal face 70 of base 58 engages a stop 72 of head 44 defined by the inner surface of head 44 and extending transverse to axis F, as shown in FIG. 15, to prevent axial translation of base 58 relative to head 44, in the direction shown by arrow G. Stop 72 is positioned below cavity 50 such that face 70 is positioned distal of cavity 50 when thread form 60 fully engages the thread form on portion 64. This configuration permits engagement of head 44 with base 58, without reducing the stiffness of head 44. In some embodiments, face 70 and/or stop 72 may be disposed at alternate orientations relative to axis F, such as, for example, transverse, perpendicular and/or other angular orientations such as acute or obtuse.

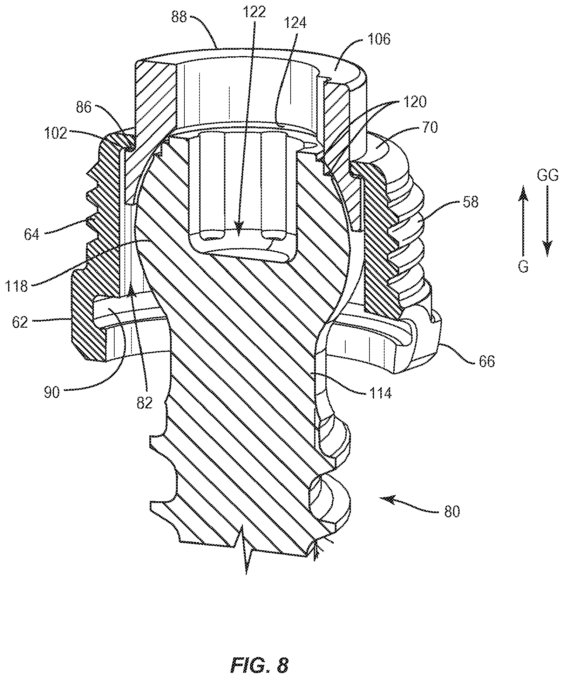

Portion 66 defines a side cavity 74 configured for moveable disposal of a distal portion, such as, for example, a threaded shaft 80 when shaft 80 is positioned within a void 82 defined by an inner surface 84 of base 58, as shown in FIG. 8. Cavity 74 is concavely curved between a first end 76 and a second end 78. In some embodiments, cavity 74 may be variously configured and dimensioned, such as, for example, convex, polygonal, irregular, uniform, non-uniform, staggered, tapered, consistent or variable.

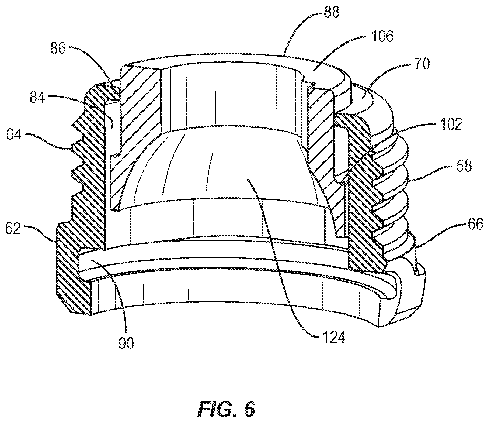

Surface 84 and face 70 define a circumferential flange 86, as shown in FIG. 6, extending transverse to axis F. Surface 84 includes a plurality of flats that define a first mating surface configured to engage a second mating surface of a second member, such as, for example, a crown 88, as shown in FIG. 4. Surface 84 defines a plurality of planar surfaces such that at least a portion of surface 84 has a hexagonal configuration. In some embodiments, surface 84 may include one or a plurality of flats.

Base 58 includes a groove 90, as shown in FIG. 6, extending into surface 84, in a direction transverse to axis F. Groove 90 includes a concave or semi-cylindrical configuration for disposal of a third member, such as, for example, a ring 92 to maintain shaft 80 within void 82. Shaft 80 is moveably disposed within cavity 74 such that a distal end of shaft 80 is disposed for movement in a range of greater than 0 degrees through 45 degrees relative to axis F. In some embodiments, groove 90 may be disposed at alternate orientations, relative to axis F, such as, for example, perpendicular and/or other angular orientations such as acute or obtuse, coaxial and/or may be offset or staggered. In some embodiments, all or only a portion of groove 90 may have various surface configurations, such as, for example, rough, arcuate, undulating, porous, semi-porous, dimpled, polished and/or textured, to enhance engagement of groove 90 with ring 92.

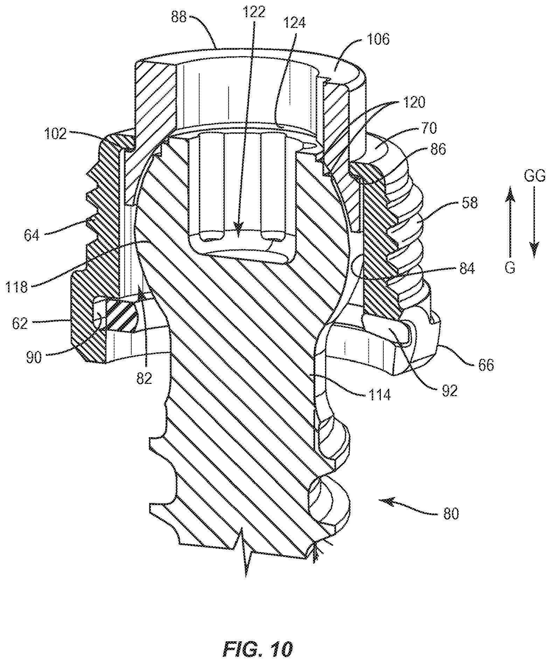

Crown 88 is configured for disposal in void 82 to fix crown 88 with base 58. Crown 88 includes a wall defining an inner surface 94 and an outer surface 96. Surface 94 defines a passageway configured for disposal of a tool configured to engage shaft 80, as will be discussed. Surface 96 includes a first portion 98 and a second portion 100 each extending parallel to axis F. Portion 100 has a width that is greater than a width of portion 98. An interface between portion 98 and portion 100 defines a second mating surface 102 having a plurality of flats and extending transverse to axis F. Surface 102 is configured to engage flange 86 when crown 88 is disposed within void 82, as shown in FIGS. 8 and 10, to prevent axial translation of crown 88 within void 82, in the direction shown by arrow G. When surface 102 engages flange 86, a proximal face 106 of crown 88 is positioned above face 70. In some embodiments, surface 102 may be disposed at alternate orientations, relative to axis F, such as, for example, perpendicular and/or other angular orientations such as acute or obtuse, coaxial and/or may be offset or staggered.

Portion 100 includes a plurality of planar surfaces defining a hexagonal configuration configured to align with the planar surfaces that define the hexagonal configuration of surface 84 to prevent rotation of crown 88 relative to base 58 about axis F, in the direction shown by arrow H in FIG. 1, or, in the direction shown by arrow HH. Crown 88 includes a key connection 104 having a polygonal configuration extending from surface 94, in a direction that is transverse to axis F. Connection 104 is configured for disposal of a tool to facilitate simultaneous rotation of crown 88 and base 58 relative to head 44. In some embodiments, surface 84, portion 100 and/or portion 104 may be variously configured and dimensioned, such as, for example, planar, concave, convex, polygonal, irregular, uniform, non-uniform, staggered, tapered, consistent or variable.

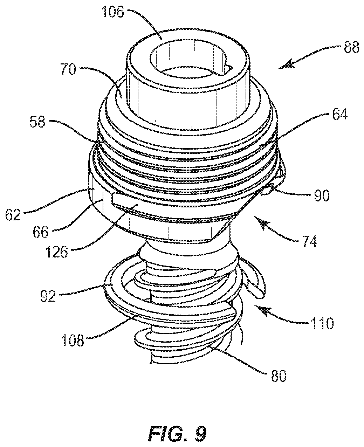

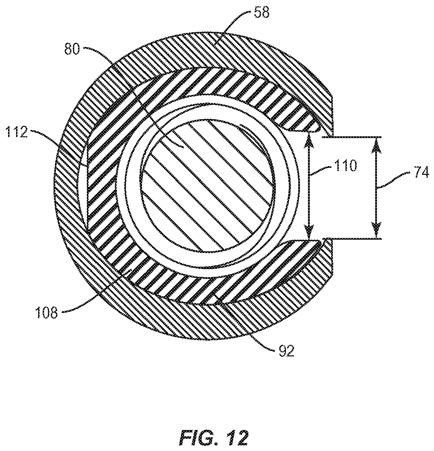

Ring 92 includes a C-shaped band portion 108 extending between a first end and a second end. The first and second ends of portion 108 are spaced apart by an opening 110, as shown in FIGS. 9 and 12. Portion 108 is configured to engage an outer surface of shaft 80 and is disposable with groove 90 to prevent axial translation of shaft 80 relative to base 58, in the direction shown by arrow GG. Opening 110 is aligned with cavity 74 when portion 108 engages an outer surface of shaft 80, as shown in FIG. 12, to permit shaft 80 to be moveably disposed within cavity 74 and void 82 such that a distal end of shaft 80 is disposed in a range of greater than 0 degrees through 45 degrees relative to axis F. In one embodiment, groove 90 has a circular configuration and ring 92 has an elliptical ring configuration such that the shape of ring 92 maintains alignment of opening 110 with cavity 74. In one embodiment, as shown in FIG. 12, ring 92 has a U-shaped configuration that includes a planar portion 112 opposite opening 110 engageable with a tool to rotate ring 92 relative to base 58 to align opening 110 with cavity 74. In some embodiments, groove 90 and/or ring 92 may have alternate cross section configurations, such as, for example, oval, oblong, triangular, square, hexagonal, polygonal, irregular, uniform, non-uniform and/or tapered.

Shaft 80 includes a first end 114 disposed within base 58 and a threaded second end 116, as shown in FIG. 2, configured to penetrate tissue, such as, for example, bone. End 114 includes a substantially spherical proximal portion 118 configured for engagement with crown 88 and moveable disposal within void 82 and cavity 74. Portion 118 includes a plurality of ridges 120 to improve purchase of portion 118 with crown 88. A lower surface 124 of crown 88 is concave or semi-spherical to accommodate the substantially spherical configuration of portion 118 such that head 44 is rotatable relative to shaft 80. This configuration allows end 116 to be rotatable relative to axis F. Head 44 is rotatable relative to shaft 80 about axis F in a caudal or cephalad orientation in a range of 0-180 degrees. In one embodiment, head 44 is rotatable relative to shaft 80 to a selected angle through and within angular range α about axis F.

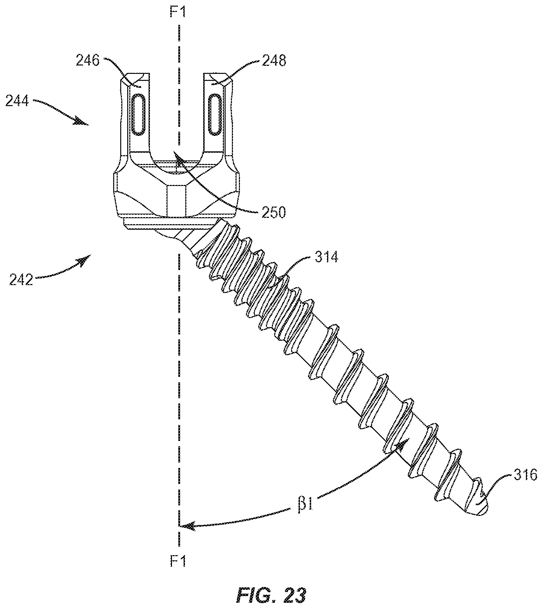

End 116 is movable relative to head 44 between a first orientation in which shaft 80 is coaxial with axis F, as shown in FIG. 1, and a second orientation in which end 116 is disposed in an angular range, for example angular range β, of greater than 0 degrees through about 45 degrees relative to axis F (see, for example, bone fastener 242 shown in FIG. 23). In some embodiments, in the second orientation, at least a portion of portion 118 is disposed in cavity 74. In one embodiment, end 116 is rotatable to a selected angle through and within angular range β relative to axis F. In some embodiments, end 116 is rotatable in a plurality of planes that lie in a cone configuration about head 44 that defines a range of motion of end 116 about axis F. In one embodiment, end 116 is rotatable to a selected angle within angular range β in a sagittal plane of a body of a patient. In one embodiment, end 116 is rotatable to a selected angle within angular range β in a transverse plane of the body. In one embodiment, end 116 is rotatable to a selected angle within angular range β in a coronal plane of the body.

End 114 includes a socket 122 having a hexalobe geometry configured for disposal of a similarly shaped bit of a tool, such as, for example, a driver to engage the driver with shaft 80 to rotate shaft 80, in the direction shown by arrow G, or, in the arrow GG. Socket 122 is in communication with opening 56, void 82 and the opening in crown 88 defined by surface 94, as shown in FIG. 4, such that a driver may be inserted between arms 46, 48 and translated axially, in the direction shown by arrow GG, until the bit of the driver is disposed in socket 122. In some embodiments, socket 122 has a cruciform, phillips, square, hexagonal, polygonal, star or hexalobe cross sectional configuration configured for disposal of a correspondingly shaped portion of a driver.

In some embodiments, at least a portion of head 44 is deformable and defines a first staked zone 128. In one embodiment, zone 128 includes a circular recess extending transverse to axis F in an outer surface of head 44. In one embodiment, zone 128 is flush with the outer surface of head 44. In some embodiments, head 44 may include one or a plurality of zones 128.

Portion 66 includes a channel 126 extending into surface 62, in a direction transverse to axis F. Channel 126 defines a second staked zone configured for engagement with zone 128. When thread form 60 fully engages the thread form on portion 64, zone 128 engages the zone of channel 126. Head 44 is staked through zone 128 to fill the channel 126 with the staked material. This configuration prevents disassembly of head 44 and base 58. In one embodiment, channel 126 extends around substantially the entire circumference of portion 66 and is interrupted by cavity 74.

In one embodiment, the components of fastener 42 are assembled. Crown 88 is positioned distal to void 82 leading with portion 98 such that the hexagonal configuration of surface 96 is aligned with the hexagonal configuration of surface 84. Connection 104 is aligned with cavity 74. Crown 88 is slid into base 58 and translated axially until surface 102 engages surface 84. In one embodiment, surfaces 102, 84 interact such that base 58 can be rotated from a top of fastener 42. For example, an instrument can engage connection 104 and spin the fastener sub-assembly to orient cavity 74 in a selected plane.

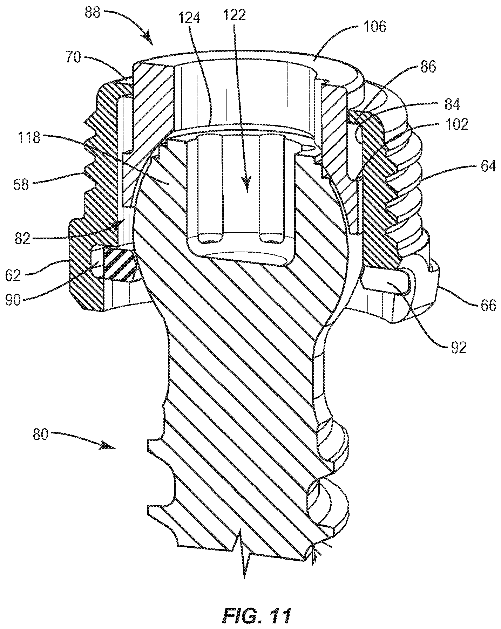

End 114 is positioned in void 82 and translated axially, in the direction shown by arrow G in FIG. 8, such that portion 118 engages surface 124. This configuration disposes crown 88 in an up position, as shown in FIG. 8. Ring 92 is positioned about shaft 80, as shown in FIG. 9, with opening 110 aligned with cavity 74. Ring 92 is inserted and/or snapped into groove 90, as shown in FIG. 10. The end 114/crown 88 sub-assembly is slid axially, in the direction shown by arrow GG, within base 58 to a down position, as shown in FIG. 11, such that end 114 engages ring 92 with opening 110 aligned with cavity 74.

The base 58 sub-assembly is threaded with head 44. Thread form 60 is aligned with the thread form on portion 64. Head 44 is rotated, in the direction shown by arrow H or the direction shown by arrow HH in FIG. 1, causing head 44 to translate axially relative to base 58 such that face 70 engages stop 72. Head 44 is threadably fixed with the base 58 sub-assembly. The staked zones, described herein, engage to prevent disassembly of head 44 and base 58.

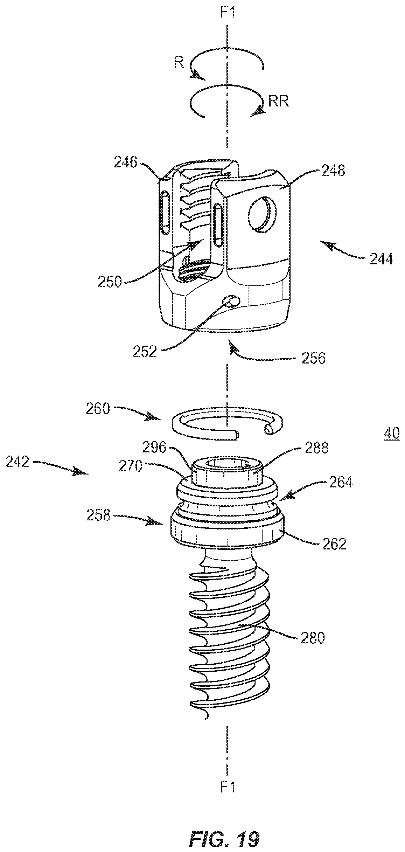

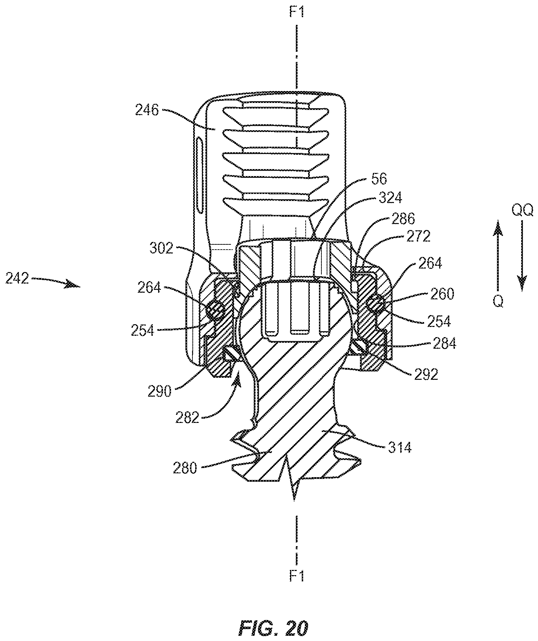

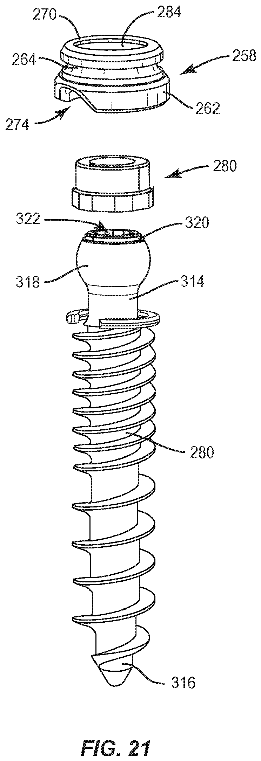

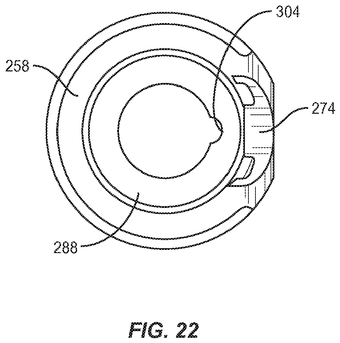

In one embodiment, as shown in FIGS. 19-24, system 40, similar to the systems and methods described above with regard to FIGS. 1-18, includes a bone fastener 242, similar to fastener 42. Fastener 242 includes a head 244, similar to head 44, extending along an axis F1 and including arms 246, 248 that define an implant cavity 250. An inner surface of head 244 defines an opening 256 configured for disposal of a base 258, similar to base 58. The inner surface of head 244 includes an inner circumferential cavity 254, as shown in FIG. 20, configured for disposal of a fourth member, such as, for example, a wire 260 that attaches head 244 with base 258. Cavity 254 extends transverse to axis F1 and has a concave or semi-cylindrical configuration. In some embodiments, the cross section surfaces defining cavity 254 may be variously configured and dimensioned, such as, for example, planar, convex, polygonal, irregular, uniform, non-uniform, staggered, tapered, consistent or variable. In some embodiments, all or only a portion of cavity 254 may have various surface configurations, such as, for example, rough, threaded, arcuate, undulating, porous, semi-porous, dimpled, polished and/or textured, to enhance fixation of wire 260 with head 244.

An outer surface of head 244 includes an aperture 252 extending between and through the inner and outer surfaces of head 244. Aperture 252 is in communication with cavity 254 such that wire 260 can be inserted through the outer surface of head 244 and into cavity 254. In some embodiments, head 244 may include one or a plurality of apertures.

Base 258 is configured for fixation with head 244 and includes a wall having a lateral surface 262 including an outer circumferential cavity 264, as shown in FIG. 19. Cavity 264 extends transverse to axis F1 into surface 262 and has a concave or semi-cylindrical configuration. In some embodiments, the cross section surfaces defining cavity 264 may be variously configured and dimensioned, such as, for example, planar, convex, polygonal, irregular, uniform, non-uniform, staggered, tapered, consistent or variable. In some embodiments, all or only a portion of cavity 264 may have various surface configurations, such as, for example, rough, threaded, arcuate, undulating, porous, semi-porous, dimpled, polished and/or textured, to enhance fixation of wire 260 with base 258.

Base 258 is disposed within opening 256 such that cavity 254 is aligned with cavity 264 and cavity 264 is in communication with aperture 252. Wire 260 is inserted through aperture 252 and into cavities 254, 264 to fix head 244 with base 258. In one embodiment, wire 260 includes a nitinol wire. In some embodiments, wire 260 may be made from a deformable material such that wire 260 may have a straight or linear configuration when wire 260 inserted through aperture 252 for disposal in cavities 254, 264 and then deform such that wire 260 has a C-shaped configuration when disposed in cavities 254, 264.

When cavities 254, 264 are aligned and wire 260 is disposed within cavities 254, 264, a proximal face 270 of base engages a stop 272 of head 244 defined by the inner surface of head 244 and extending transverse to axis F1, as shown in FIG. 20, to prevent axial translation of base 258 relative to head 244, in the direction shown by arrow Q. Stop 272 is positioned below cavity 250 such that face 270 is positioned distal of cavity 250 when cavities 254, 264 are aligned and wire 260 is disposed within cavities 254, 264. This configuration permits engagement of head 244 with base 258, without reducing the stiffness of head 244.

Surface 262 includes a cavity 274, similar to cavity 74, configured for moveable disposal of a threaded shaft 280, similar to shaft 80, when shaft 280 is positioned within a void 282 defined by an inner surface 286 of base 258. An inner surface 284 of base 258 defines a circumferential flange 286 extending transverse to axis F1. Surface 284 includes a single flat that defines a first mating surface configured to engage a second mating surface of a crown 288, similar to crown 88.

Base 258 includes a groove 290, similar to groove 90, extending into surface 284, in a direction that is transverse to axis F1, and configured for disposal of a ring 292, similar to ring 92. Ring 292 maintains shaft 280 within a void 282 defined by surface 284 such that shaft 280 is moveably disposed within cavity 274 and a distal end of shaft 280 is disposed for movement in a range of greater than 0 degrees through 45 degrees relative to axis F1.

Crown 288 is configured for disposal in void 282 to fix crown 288 with base 258. Crown 288 includes a wall defining an inner surface 294 and an outer surface 296. Surface 294 defines a passageway configured for disposal of a tool configured to engage shaft 280. Surface 296 includes an interface defining a second mating surface 302 having a single flat and extending transverse to axis F1. Surface 302 is configured to engage surface 284 when crown 288 is disposed within void 282 to prevent axial translation of crown 288 within void 282, in the direction shown by arrow Q.

Ring 292 engages an outer surface of shaft 280 and is disposable with groove 290 to prevent axial translation of shaft 280 relative to base 258, in the direction shown by arrow QQ. The opening in ring 292 is aligned with cavity 274 when ring 292 engages an outer surface of shaft 280 to permit shaft 280 to be moveably disposed within cavity 274 and void 282 such that a distal end of shaft 280 is disposed for movement in a range of greater than 0 degrees through 45 degrees relative to axis F1.

Shaft 280 includes a first end 314 disposed within base 258 and a threaded second end 316 configured to penetrate tissue, such as, for example, bone. End 314 includes a substantially spherical proximal portion 318 configured for engagement with crown 288 and moveable disposal within void 282 and cavity 274. Portion 318 includes a plurality of ridges 320 to improve purchase of portion 318 with crown 288. A lower surface 324 of crown 288 is concave or semi-spherical to accommodate the substantially spherical configuration of portion 318 such that head 244 is rotatable relative to shaft 280. This configuration also allows end 316 to be rotatable relative to axis F1.

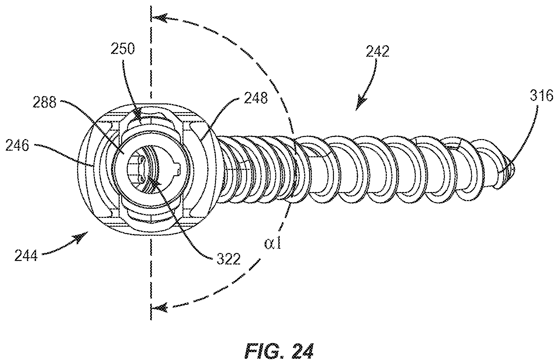

Head 244 is rotatable relative to shaft 280 in a caudal orientation in a range of 0-180 degrees. In one embodiment, head 244 is rotatable relative to shaft 280 to a selected angle through and within angular range α1 about axis F1, as shown in FIG. 24. End 316 is movable relative to head 244 between a first orientation, described above with regard to FIG. 1, and a second orientation in which end 316 is disposed in an angular range, for example angular range β1, of greater than 0 degrees through about 45 degrees relative to axis F1, as shown in FIG. 23. In some embodiments, in the second orientation, at least a portion of portion 318 is disposed in cavity 274. In one embodiment, end 316 is rotatable to a selected angle through and within angular range β1 relative to axis F1. In some embodiments, end 316 is rotatable in a plurality of planes that lie in a cone configuration about head 244 that defines a range of motion of end 316 about axis F1. In one embodiment, end 316 is rotatable to a selected angle within angular range β1 in a sagittal plane of a body of a patient. In one embodiment, end 316 is rotatable to a selected angle within angular range β1 in a transverse plane of the body. In one embodiment, end 316 is rotatable to a selected angle within angular range β1 in a coronal plane of the body.

End 314 includes a socket 322 having a hexalobe geometry configured for disposal of a similarly shaped bit of a tool, such as, for example, a driver to engage the driver with shaft 280 to rotate shaft 280. Socket 322 is in communication with opening 256, void 282 and the opening in crown 288 defined by surface 294 such that a driver may be inserted between arms 246, 248 and translated axially, in the direction shown by arrow QQ, until the bit of the driver is disposed in socket 322.

In one embodiment, the components of fastener 242 are assembled. Crown 288 is positioned distal to void 282 and disposed such that the single flat configuration of surface 302 is aligned with the single flat configuration of surface 284. A connection 304 of crown 288 is aligned with cavity 274. Crown 288 is slid into base 258 and translated axially until surface 302 engages surface 284. In one embodiment, surfaces 302, 284 interact such that base 258 can be rotated from a top of fastener 242. For example, an instrument can engage connection 304 and spin the fastener sub-assembly to orient cavity 274 in a selected plane.

End 314 is positioned in void 282 and translated axially such that portion 318 engages surface 324. This configuration disposes crown 288 in an up position, similar to that described with regard to FIG. 8. Ring 292 is positioned about shaft 280 with the opening of ring 292 aligned with cavity 74. Ring 292 is inserted and/or snapped into groove 290. The end 314/crown 288 sub-assembly is slid axially within base 258 to a down position, similar to that described with regard to FIG. 11, such that end 314 engages ring 292 with the opening of ring 292 aligned with cavity 274.

Base 258 is disposed within opening 256 such that cavity 254 is aligned with cavity 264 and cavity 264 is in communication with aperture 252. Wire 260 is inserted through aperture 252 and into cavities 254, 264 to fix head 244 with base 258, as described above.

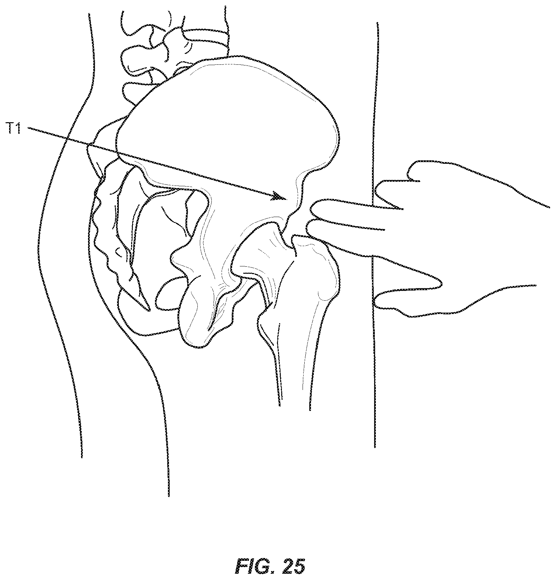

In assembly, operation and use, spinal implant system 40, similar to the systems and methods described herein, includes fastener 42 and/or bone fastener 242 and is employed with a surgical procedure for treatment of a spinal disorder affecting a section of a spine of a patient, as discussed herein. Spinal implant system 40 is employed with a surgical procedure for treatment of a condition or injury of an affected section of the spine including vertebrae V. In one embodiment, as shown in FIGS. 25 and 26, spinal implant system 40 includes fasteners 242 attached to a sacrum S for a surgical arthrodesis procedure, such as a fusion of the affected section of the spine to facilitate healing and therapeutic treatment. In one embodiment, the components of spinal implant system 40 are attached to vertebrae V including sacrum S and/or an ilium I for a dynamic stabilization application.

In use, to treat the affected section of the spine, a medical practitioner obtains access to a surgical site including vertebrae V, sacrum S and ilium I in any appropriate manner, such as through incision and retraction of tissues. In some embodiments, the components of spinal implant system 40 may be used in any existing surgical method or technique including open surgery, mini-open surgery, minimally invasive surgery including percutaneous surgical implantation, whereby vertebrae V, sacrum S and/or ilium I are each accessed through a micro-incision, or sleeve that provides a protected passageway to the area. Once access to the surgical site(s) is obtained, the particular surgical procedure is performed for treating the spinal disorder. The components of spinal implant system 40 including fasteners 242 are employed to augment the surgical treatment. Fasteners 242 and one or a plurality of spinal implants, such as, for example, vertebral rods 130 can be delivered or implanted as a pre-assembled device or can be assembled in situ. Spinal implant system 40 may be may be completely or partially revised, removed or replaced.

Spinal implant system 40 includes two spinal rods 130, which are disposed in an axially aligned and spaced apart configuration, that extend to adjacent a sacroiliac region of the patient. Spinal rods 130 each have a rigid, arcuate portion 132 extending across sacrum S of the sacroiliac region. A first fastener 242 and a second fastener 242 are configured for fixation with spaced apart regions of sacrum S. The first fastener 242 is positioned adjacent a first selected region of sacrum S and the second fastener 242 is positioned adjacent a second selected region of sacrum S.

Pilot holes are drilled at a selected trajectory T, each of which is oriented along a S2-alar-iliac trajectory T, as shown in FIG. 25. For example, a pilot hole is initially tapped in the first selected region of sacrum S and then extended through sacrum S and ilium I along a trajectory T1. The pilot hole is oriented for disposal of first fastener 242 such that first fastener 242 may be attached with the first selected region of sacrum S and ilium I along trajectory T1.

A pilot hole is initially tapped in the second selected region of sacrum S and then extended through sacrum S and ilium I along a trajectory T2. The pilot hole is oriented for disposal of second fastener 242 such that second fastener 242 may be attached with the second selected region of sacrum S and ilium I along trajectory T1. In one embodiment, the selected trajectory is within angular range of about 0 degrees to about 45 degrees relative to an outer surface of sacrum S. In some embodiments, only a single selected region of sacrum S is fixed with one or a plurality of fasteners 242.

An instrument, such as, for example, a driver is inserted between arms 246, 248 of the first fastener 242 and engaged with socket 122. The driver is rotated, in the direction shown by arrow R in FIG. 19, causing the first fastener 242 to translate axially within the first pilot hole, in the direction shown by arrow QQ in FIG. 20. Shaft 280 is threaded with sacrum S and ilium I along trajectory T1 to attach the first fastener 242 with the sacroiliac region adjacent the first selected region of sacrum S.

With shaft 280 fixed with sacrum S and ilium I along trajectory T1, cavity 250 of head 244 is selectively rotatable relative to shaft 280, as described herein, to a selected angle through and within angular range α1 relative to axis F1, as shown in FIG. 24. This configuration facilitates alignment of cavity 250 with portion 132 for disposal of portion within cavity 250. Cavity 250 is relatively rotatable to receive, engage and accommodate the orientation and position of portion 132 of the first spinal rod 130.

The first spinal rod 130 is positioned within cavity 250 of the first fastener 242. A coupling element, such as, for example, a setscrew may be torqued and threaded with head 244 to securely attach the first spinal rod 130 with the sacroiliac region adjacent the first selected region of sacrum S.

A driver is inserted between arms 246, 248 of the second fastener 242 and engaged with socket 122. The driver is rotated, in the direction shown by arrow R in FIG. 19, causing the second fastener 242 to translate axially within the second pilot hole, in the direction shown by arrow QQ in FIG. 20. Shaft 280 is threaded with sacrum S and ilium I along trajectory T2 to attach the second fastener 242 with the sacroiliac region adjacent the second selected region of sacrum S.

With shaft 280 fixed with sacrum S and ilium I along trajectory T2, cavity 250 of head 244 is selectively rotatable relative to shaft 280, as described herein, to a selected angle through and within angular range α1 relative to axis F1, as shown in FIG. 24. This configuration facilitates alignment of cavity 250 with portion 132 for disposal of portion within cavity 250. Cavity 250 is relatively rotatable to receive, engage and accommodate the orientation and position of portion 132 of the second spinal rod 130.

The second spinal rod 130 is positioned within cavity 250 of the second fastener 242. A setscrew may be torqued and threaded with head 244 to securely attach the second spinal rod 130 with the sacroiliac region adjacent the second selected region of sacrum S.

In some embodiments, spinal implant system 40 includes an agent, which may be disposed, packed, coated or layered within, on or about the components and/or surfaces of spinal implant system 40. In some embodiments, the agent may include bone growth promoting material, such as, for example, bone graft to enhance fixation of the fixation elements with vertebrae V.

In some embodiments, the agent may include therapeutic polynucleotides or polypeptides. In some embodiments, the agent may include biocompatible materials, such as, for example, biocompatible metals and/or rigid polymers, such as, titanium elements, metal powders of titanium or titanium compositions, sterile bone materials, such as allograft or xenograft materials, synthetic bone materials such as coral and calcium compositions, such as HA, calcium phosphate and calcium sulfite, biologically active agents, for example, gradual release compositions such as by blending in a bioresorbable polymer that releases the biologically active agent or agents in an appropriate time dependent fashion as the polymer degrades within the patient. Suitable biologically active agents include, for example, BMP, Growth and Differentiation Factors proteins (GDF) and cytokines. The components of spinal implant system 40 can be made of radiolucent materials such as polymers. Radiomarkers may be included for identification under x-ray, fluoroscopy, CT or other imaging techniques. In some embodiments, the agent may include one or a plurality of therapeutic agents and/or pharmacological agents for release, including sustained release, to treat, for example, pain, inflammation and degeneration.

In some embodiments, the use of microsurgical and image guided technologies may be employed to access, view and repair spinal deterioration or damage, with the aid of spinal implant system 40. Upon completion of the procedure, the surgical instruments, assemblies and non-implant components of spinal implant system 40 are removed from the surgical site and the incision is closed.

In some embodiments, spinal implant system 40 can include one or a plurality of fasteners 42, 242, such as those described herein and/or fixation elements, which may be employed with a single vertebral level or a plurality of vertebral levels. In some embodiments, fasteners 42, 242 may be engaged with vertebrae in various orientations, such as, for example, series, parallel, offset, staggered and/or alternate vertebral levels. In some embodiments, fasteners 42, 242 may be configured as multi-axial screws, sagittal angulation screws, pedicle screws, mono-axial screws, uni-planar screws, fixed screws, anchors, tissue penetrating screws, conventional screws, expanding screws. In some embodiments, fasteners 42, 242 may be employed with wedges, anchors, buttons, clips, snaps, friction fittings, compressive fittings, expanding rivets, staples, nails, adhesives, posts, connectors, fixation plates and/or posts.

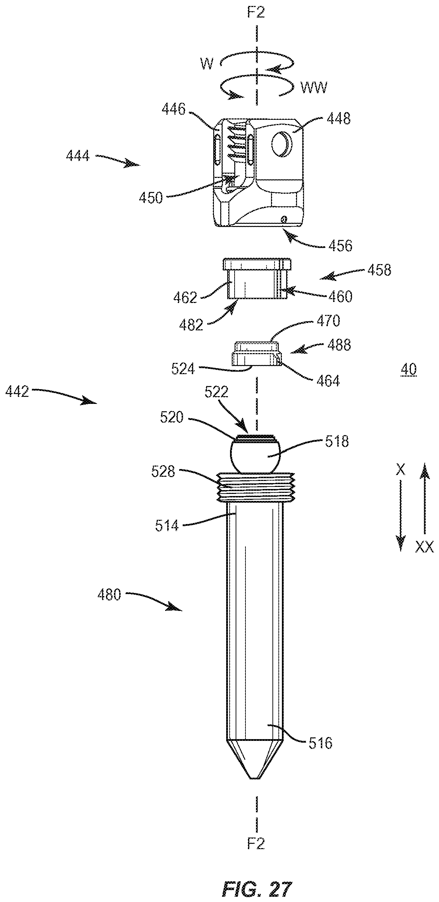

In one embodiment, as shown in FIG. 27, system 40, similar to the systems and methods described herein, includes a bone fastener 442, similar to fasteners 42, 242. Fastener 442 includes a head 444 extending along an axis F2. Head 444 includes a pair of spaced apart arms 446, 448 that define a U-shaped implant cavity 450 therebetween configured for disposal of a spinal construct, such as, for example, rod 130.

An inner surface of head 444 defines an opening 456 configured for disposal of a base 458. At least a portion of the inner surface of head 444 is threaded. In one embodiment, the inner surface of head 444 is threaded adjacent opening 456. Base 458 is configured for fixation with head 444 and includes a wall including an outer surface 462 configured to engage the inner surface of head 444. Surface 462 includes a slot 460 extending parallel to axis F2. The wall also includes an inner surface defining a void 482 configured for disposal of a crown 488.

Crown 488 includes a wall defining an inner surface and an outer surface 496 configured to engage the inner surface of base 458. Surface 496 includes a projection 464 extending from surface 496, in a direction that is transverse to axis F2. When crown 488 is disposed in void 482, projection 464 is disposed within slot 460 to prevent rotation of crown 488 about axis F2 relative to base 458, in a direction shown by arrow W or arrow WW. The inner surface of crown 488 defines a passageway configured for disposal of a tool configured to engage a shaft 480, as will be discussed.

Shaft 480 includes a first end 514 disposed within base 458 and a second end 516 configured to penetrate tissue, such as, for example, bone. End 514 includes a substantially spherical proximal portion 518 configured for engagement with crown 488 and moveable disposal within void 482. Portion 518 includes a plurality of ridges 520 to improve purchase of portion 518 with crown 588. A lower surface 524 of crown 488 is concave or semi-spherical to accommodate the substantially spherical configuration of portion 518 such that a planar face 470 of crown 488 is rotatable relative to shaft 480. This configuration also allows face 470 to be rotatable relative to axis F2 such that face 470 can move to accommodate the orientation of an implant, such as, for example, rod 130 positioned in cavity 450. End 514 includes a threaded portion 528 configured to engage the threaded portion of the inner surface of head 444 to engage shaft 480 with head 444.

In one embodiment, face 470 is rotatable to a selected angle through and within angular range relative to axis F2, similar to that described above. End 514 includes a socket 522 having a hexalobe geometry configured for disposal of a similarly shaped bit of a tool, such as, for example, a driver to engage the driver with shaft 480 to rotate shaft 480, in the direction shown by arrow W or the arrow WW. Socket 422 is in communication with opening 456, void 482 and an opening in crown 488 defined an inner surface of crown 488 such that a driver may be inserted between arms 446, 448 and translated axially in the direction shown by arrow X until the bit of the driver is disposed in socket 522.

To assemble the components of fastener 442, crown 488 is positioned distal to void 482. Projection 464 is aligned with slot 460 and crown 488 is translated axially, in the direction shown by arrow XX, such that projection 464 is disposed within slot 460 to prevent rotation of crown 488 relative to base 458. End 514 is positioned in void 482 and translated axially, in the direction shown by arrow XX, until portion 518 engages surface 524. Head 444 is positioned relative to shaft 480 such that the threads in the inner surface of head 444 are aligned with the threads of portion 528. Head 444 is rotated relative to axis F2, in the direction shown by arrow W or arrow WW, such that head 444 translates axially relative to shaft 480, in the direction shown by arrow X.

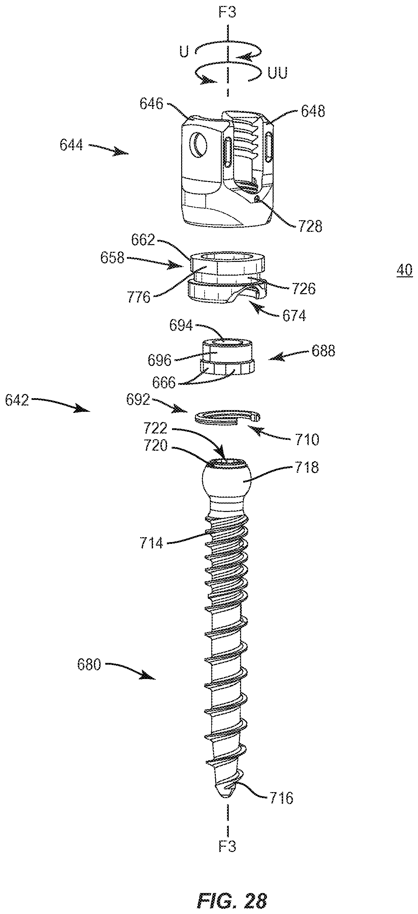

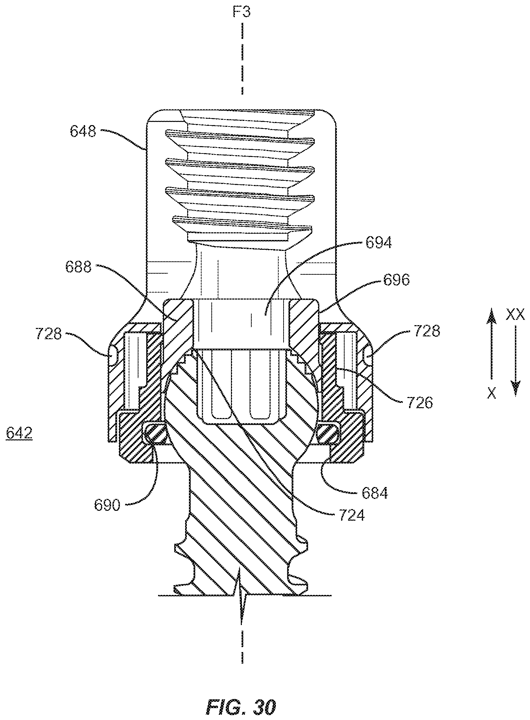

In one embodiment, as shown in FIGS. 28-30, system 40, similar to the systems and methods described herein, includes a bone fastener 642, similar to the fasteners described above. Fastener 642 includes a head 644 extending along an axis F3. Head 644 includes a pair of spaced apart arms 646, 648 that define a U-shaped implant cavity 650 therebetween configured for disposal of rod 130. An inner surface of head 644 defines an opening 656 configured for disposal of a base 658. The inner surface of head 644 includes a circumferential flange 676 extending transverse to axis F3 from the inner surface of head 644. Flange 676 includes one or a plurality of mating surfaces, such as, for example, protrusions 678 configured to engage an outer surface of base 658 to prevent disassembly of base 658 from head 644.

At least a portion of head 644 is deformable and defines a staked zone 728. In one embodiment, head 644 includes a pair of zones 728 on opposite portions of head 644. In one embodiment, zones 728 are positioned proximal to flange 676. In one embodiment, zones 728 are positioned distal to flange 676.

Base 658 is configured for fixation with head 644 and includes a wall having a lateral surface 662 including a cavity 674 configured for moveable disposal of a threaded shaft 680 when shaft 680 is positioned within a void 682 defined by an inner surface 686 of base 658. Cavity 674 is concavely curved between a first end and a second end.

Surface 662 defines a mating surface, such as, for example, a circumferential channel 726 configured for disposal of protrusions 678 and/or zone 728. Head 644 is positioned relative to base 658 such that protrusions 678 and zones 728 are aligned with channel 726. Zones 728 are contacted with a tool, such as, for example, a centerpunch such that zones 728 translate toward axis F3 and are disposed in channel 726 to prevent disassembly of head 644 and base 658.

Surface 662 includes planar surfaces 776 configured to allow protrusions 678 to slide therealong to facilitate assembly of head 644 with base 658. Upon disposal of protrusions 678 with channel 726, head 644 can be rotated relative to base 658 such that protrusions 678 are rotated within channel 726. Protrusions 678 are rotated out of alignment with surfaces 776 such that surface 662 provides an interference and prevents protrusions 678 from passing out of channel 726. Surface 662 provides an interference with flange 676 such that head 644 is interference fit with base 658.

Base 658 includes a groove 690 extending into an inner surface 684. Groove 690 is concave or semi-cylindrical and is configured for disposal of a ring 692 to maintain shaft 680 within void 682 such that shaft 680 is moveably disposed within cavity 674 and a distal end of shaft 680 is disposed for movement in a range of greater than 0 degrees through 45 degrees, similar to that described herein. Surface 684 defines a plurality of planar surfaces such that surface 684 has a hexagonal configuration.

A crown 688, similar to those described herein, is configured for disposal in void 682 to fix crown 688 with base 658. Crown 688 includes a wall defining an inner surface 694 and an outer surface 696. Surface 694 defines a passageway configured for disposal of a tool configured to engage shaft 680. Surface 696 includes a plurality of planar surfaces 666 defining a hexagonal configuration configured to align with the planar surfaces that define the hexagonal configuration of surface 684 to prevent rotation of crown 688 relative to base 658 about axis F3.

Ring 692 includes a C-shaped band portion extending between a first end and a second end. The first and second ends of the band portion of ring 692 are spaced apart by an opening 710. Ring 692 is configured to engage an outer surface of shaft 680 and is disposable with groove 690 to prevent axial translation of shaft 680 relative to base 658. Opening 710 is aligned with cavity 674 when ring 692 engages an outer surface of shaft 680 to permit shaft 680 to be moveably disposed within cavity 674 and void 682 such that a distal end of shaft 680 is disposed for movement in a range of greater than 0 degrees through 45 degrees relative to axis F3.

Shaft 680 includes a first end 714 disposed within base 658 and a threaded second end 716 configured to penetrate tissue, such as, for example, bone. End 714 includes a substantially spherical proximal portion 718 configured for engagement with crown 688 and moveable disposal within void 682 and cavity 674. Portion 718 includes a plurality of ridges 720 to improve purchase of portion 718 with crown 688. A lower surface 724 of crown 688 is concave or semi-spherical to accommodate the substantially spherical configuration of portion 718 such that head 644 is rotatable relative to shaft 680. This configuration also allows end 716 to be rotatable relative to axis F3.

Head 644 is rotatable relative to shaft 680 in a caudal orientation and cephalad orientation in a range of 0-180 degrees, similar to that described herein. End 716 is movable relative to head 644 between a first orientation in which shaft 680 is coaxial with axis F3 and a second orientation in which end 716 is disposed in a range of greater than 0 degrees through 45 degrees relative to axis F3 and at least a portion of portion 718 is disposed in cavity 674.

End 714 includes a socket 722 having a hexalobe geometry configured for disposal of a similarly shaped bit of a tool, such as, for example, a driver to engage the driver with shaft 680 to rotate shaft 680 about axis F3 in a clockwise or counterclockwise direction.

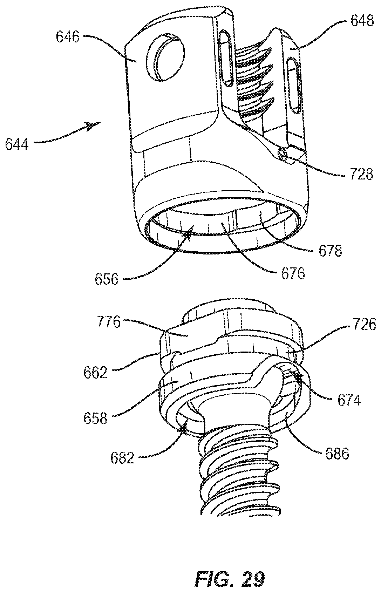

To assemble the components of fastener 642, crown 688 is positioned in void 682 such that the hexagonal configuration of surface 696 is aligned with the hexagonal configuration of surface 684. Crown 688 is translated axially, in the direction shown by arrow Y in FIG. 29. End 714 is positioned in void 682 and translated axially, in the direction shown by arrow Y, until portion 718 engages surface 724. Ring 692 is positioned about shaft 680 and is inserted into groove 690. Surfaces 776 are aligned with protrusions 678 such that protrusions 678 are disposed within channel 726 to prevent disassembly of head 644 from base 658. Zone 728 is contacted with a tool, such as, for example, a centerpunch such that zone 728 is staked with base 658 to prevent disassembly of head 644 from base 658.

It will be understood that various modifications may be made to the embodiments disclosed herein. Therefore, the above description should not be construed as limiting, but merely as exemplification of the various embodiments. Those skilled in the art will envision other modifications within the scope of the claims appended hereto.

## Claims

1. A bone fastener (42; 242; 442; 642) comprising:
a head (44; 244; 444; 644) extending along an axis (F; F1; F2; F3) and including an inner surface defining an implant cavity (50; 250; 450; 650);
a base (58; 258; 458; 658) configured for fixation with the head (44; 244; 444; 644) and including a wall, the wall defining a groove (90; 290; 690) and including an inner surface (84; 284; 684) that defines a first mating surface, the wall further including a lateral surface that defines a cavity (74; 274; 674);
a crown (88; 288; 488; 688) configured for fixation with the base (58; 258; 458; 658) and including a wall, the wall including a second mating surface (102; 302) engageable with the first mating surface;
a ring (92; 292; 692) having a band portion (108) that defines an opening (110; 710), and
a shaft (80; 280; 480; 680) including a first end (114; 314; 514; 714) disposed within the base (58; 258; 458; 658) and a second end (116; 316; 516; 716) configured to penetrate tissue,
**characterized in that** the band portion (108) is disposable with the groove (90;290;690) such that the opening (110; 710) is aligned with the cavity (74; 274; 674); and wherein the band portion (108) of the ring (92; 292; 692) is configured to engage an outer surface of the shaft (80; 280; 680) to prevent axial translation of shaft (80; 280; 680) relative to base (58; 258; 458; 658), and
wherein the opening (110; 710) is aligned with the cavity (74; 274; 674) when the band portion (108) engages the outer surface of shaft (80; 280; 680) to permit the shaft (80; 280; 680) to be moved relative to the head (44; 244; 644) between a first orientation and a second orientation such that at least a portion of the shaft (80; 280; 680) is disposed in the cavity (74; 274; 674).

2. A bone fastener (242) as recited in Claim 1, wherein the head (244) includes a wall defining an inner circumferential cavity (254) and the wall of the base (258) defines an outer circumferential cavity (264), the circumferential cavities being aligned and configured for disposal of a wire (260) in a configuration to fix the head (244) with the base (258).

3. A bone fastener (242) as recited in Claim 2, wherein the wire (260) comprises a nitinol wire.

4. A bone fastener (42) as recited in Claim 1, wherein the head (44) includes a wall having a threaded inner surface (60) and the wall of the base (58) includes a threaded outer surface (64) engageable to fix the head (44) with the base (58).

5. A bone fastener (42) as recited in Claim 4, wherein engagement of the threaded surfaces are timed such that disposal of the head (44) and the base (58) in a fixed orientation aligns the implant cavity (50) in a cephalad direction and/or a caudal direction.

6. A bone fastener (42; 242) as recited in Claim 1, wherein the first mating surface includes at least one flat and the second mating surface (102; 302) includes at least one flat, the flats being aligned to fix the base (58; 258) with the crown (88, 288).

7. A bone fastener (42) as recited in Claim 1, wherein the first mating surface has a hexagonal configuration and the second mating surface (102) includes a hexagonal configuration, the hexagonal configurations being aligned to fix the base (58) with the crown (88).

8. A bone fastener (42) as recited in Claim 1, wherein the ring (92) includes an elliptical ring configuration.

9. A bone fastener (42) as recited in Claim 1, wherein the ring (92) includes a U-shaped configuration.

10. A bone fastener (42; 242; 642) as recited in Claim 1, wherein in the first orientation the shaft (80; 280; 680) is coaxial with the axis (F; F1; F3), and in the second orientation, the shaft (80; 280; 680) is disposed in a range of greater than 0 degrees through 45 degrees relative to the axis (F; F1; F3).

11. A bone fastener (42; 242; 642) as recited in Claim 1, wherein the head (44; 244; 644) is rotatable in a caudal orientation in a range of 0-360 degrees.

12. A bone fastener (42; 242; 642) as recited in Claim 1, wherein the shaft (80; 280; 680) is disposable within the cavity (74; 274; 674) along a S2-alar-iliac trajectory.

13. A bone fastener (42; 642) as recited in Claim 1, wherein the head (44; 644) includes a first staked surface and the base (58; 658) includes a second staked surface, the staked surfaces being engageable to fix the head (44; 644) with the base (58; 658).

14. A bone fastener (42; 242) as recited in Claim 1, wherein the crown (88; 288) includes an inner surface (94; 294) defining a key connection (104; 304) engageable to facilitate rotation of the crown (88; 288) relative to the base (58; 258).

## Patentansprüche

1. Ein Knochenbefestigungselement (42, 242, 442, 642), aufweisend:
einen Kopf (44, 244, 444, 644), der sich entlang einer Achse (F, F1, F2, F3) erstreckt und eine Innenfläche aufweist, die einen Implantathohlraum (50, 250, 450, 650) definiert,
eine Basis (58, 258, 458, 658), die zum Befestigen an dem Kopf (44, 244, 444, 644) konfiguriert ist und eine Wand aufweist, wobei die Wand eine Nut (90, 290, 690) definiert und eine Innenfläche (84, 284, 684) aufweist, die eine erste Gegenfläche definiert, wobei die Wand ferner eine Seitenfläche aufweist, die einen Hohlraum (74, 274, 674) definiert,
eine Krone (88, 288, 488, 688, die zum Befestigen an der Basis (58, 258, 458, 658) konfiguriert ist und eine Wand aufweist, wobei die Wand eine zweite Gegenfläche (102, 302) aufweist, die mit der ersten Gegenfläche in Eingriff bringbar ist,
einen Ring (92, 292, 692) mit einem Bandabschnitt (108), der eine Öffnung (110, 710) definiert, und
einen Schaft (80, 280, 480, 680), der ein erstes Ende (114, 314, 514, 714), das innerhalb der Basis (58, 258, 458, 658) angeordnet ist, und ein zweites Ende (116, 316, 516, 716) aufweist, das konfiguriert ist, um Gewebe zu durchdringen,
**dadurch gekennzeichnet, dass** der Bandabschnitt (108) derart mit der Nut (90, 290, 690) angeordnet werden kann, dass die Öffnung (110, 710) zu dem Hohlraum (74, 274, 674) ausgerichtet ist, und
wobei der Bandabschnitt (108) des Rings (92, 292, 692) konfiguriert ist, um mit einer Außenfläche des Schafts (80, 280, 680) in Eingriff zu sein, um eine axiale Translation des Schafts (80, 280, 680) relativ zu der Basis (58, 258, 458, 658) zu verhindern, und
wobei die Öffnung (110, 710) zu dem Hohlraum (74, 274, 674) ausgerichtet ist, wenn der Bandabschnitt (108) mit der Außenfläche des Schafts (80, 280, 680) in Eingriff ist, um zu ermöglichen, dass der Schaft (80, 280, 680) relativ zu dem Kopf (44, 244, 644) zwischen einer ersten Ausrichtung und einer zweiten Ausrichtung bewegt wird, so dass mindestens ein Abschnitt des Schafts (80, 280, 680) in dem Hohlraum (74, 274, 674) angeordnet ist.

2. Ein Knochenbefestigungselement (242) gemäß Anspruch 1, wobei der Kopf (244) eine Wand aufweist, die einen inneren Umfangshohlraum (254) definiert, und die Wand der Basis (258) einen äußeren Umfangshohlraum (264) definiert, wobei die Umfangshohlräume zum Anordnen eines Drahtes (260) in einer Konfiguration ausgerichtet und konfiguriert sind, um den Kopf (244) an der Basis (258) zu befestigen.

3. Ein Knochenbefestigungselement (242) gemäß Anspruch 2, wobei der Draht (260) einen Nitinoldraht aufweist.

4. Ein Knochenbefestigungselement (42) gemäß Anspruch 1, wobei der Kopf (44) eine Wand mit einer Innengewindefläche (60) aufweist und die Wand der Basis (58) eine Außengewindefläche (64) aufweist, die in Eingriff bringbar sind, um den Kopf (44) an der Basis (58) zu befestigen.

5. Ein Knochenbefestigungselement (42) gemäß Anspruch 4, wobei der Eingriff der Gewindeflächen zeitlich abgestimmt ist, so dass das Anordnen des Kopfes (44) und der Basis (58) in einer festen Ausrichtung den Implantathohlraum (50) in einer kephalen Richtung und/oder einer kaudalen Richtung ausrichtet.

6. Ein Knochenbefestigungselement (42, 242) gemäß Anspruch 1, wobei die erste Gegenfläche mindestens eine Abflachung aufweist und die zweite Gegenfläche (102, 302) mindestens eine Abflachung aufweist, wobei die Abflachungen ausgerichtet sind, um die Basis (58, 258) an der Krone (88, 288) zu befestigen.

7. Ein Knochenbefestigungselement (42) gemäß Anspruch 1, wobei die erste Gegenfläche eine hexagonale Konfiguration hat und die zweite Gegenfläche (102) eine hexagonale Konfiguration aufweist, wobei die hexagonalen Konfigurationen ausgerichtet sind, um die Basis (58) an der Krone (88) zu befestigen.

8. Ein Knochenbefestigungselement (42) gemäß Anspruch 1, wobei der Ring (92) eine elliptische Ringkonfiguration aufweist.

9. Ein Knochenbefestigungselement (42) gemäß Anspruch 1, wobei der Ring (92) eine U-förmige Konfiguration aufweist.

10. Ein Knochenbefestigungselement (42, 242, 642) gemäß Anspruch 1, wobei in der ersten Ausrichtung der Schaft (80, 280, 680) mit der Achse (F, F1, F3) koaxial ist, und in der zweiten Ausrichtung der Schaft (80, 280, 680) in einem Bereich von größer als 0 Grad bis 45 Grad relativ zu der Achse (F, F1, F3) angeordnet ist.

11. Ein Knochenbefestigungselement (42, 242, 642) gemäß Anspruch 1, wobei der Kopf (44, 244, 644) in einer kaudalen Ausrichtung in einem Bereich von 0-360 Grad drehbar ist.

12. Ein Knochenbefestigungselement (42, 242, 642) gemäß Anspruch 1, wobei der Schaft (80, 280, 680) innerhalb des Hohlraums (74, 274, 674) entlang einer S2-alar-iliakalen Trajektorie angeordnet werden kann.

13. Ein Knochenbefestigungselement (42, 642) gemäß Anspruch 1, wobei der Kopf (44, 644) eine erste Stützfläche aufweist und die Basis (58, 658) eine zweite Stützfläche aufweist, wobei die Stützflächen in Eingriff bringbar sind, um den Kopf (44, 644) an der Basis (58, 658) zu befestigen.

14. Ein Knochenbefestigungselement (42, 242) gemäß Anspruch 1, wobei die Krone (88, 288) eine Innenfläche (94, 294) aufweist, die eine Schlüsselverbindung (104, 304) definiert, die in Eingriff bringbar ist, um das Drehen der Krone (88, 288) relativ zu der Basis (58, 258) zu erleichtern.

## Revendications

1. Une fixation osseuse (42, 242, 442, 642), comprenant :
une tête (44, 244, 444, 644) s'étendant le long d'un axe (F, F1, F2, F3) et comprenant une surface intérieure définissant une cavité d'implant (50, 250, 450, 650),
une base (58, 258, 458, 658) configurée pour être fixée à la tête (44, 244, 444, 644) et comprenant une paroi, la paroi définissant une rainure (90, 290, 690) et comprenant une surface intérieure (84, 284, 684) qui définit une première surface d'accouplement, la paroi comprenant en outre une surface latérale définissant une cavité (74, 274, 674),
une couronne (88, 288, 488, 688) configurée pour être fixée à la base (58, 258, 458, 658) et comprenant une paroi, la paroi comprenant une deuxième surface d'accouplement (102, 302) pouvant se mettre en prise avec la première surface d'accouplement,
un anneau (92, 292, 692) présentant une partie de bande (108) qui définit une ouverture (110, 710), et
une tige (80, 280, 480, 680) comprenant une première extrémité (114, 314, 514, 714) disposée à l'intérieur de la base (58, 258, 458, 658) et une deuxième extrémité (116, 316, 516, 716) configurée pour pénétrer le tissu,
**caractérisée en ce que** la partie de bande (108) peut être disposée avec la rainure (90, 290, 690) de sorte que l'ouverture (110, 710) est alignée avec la cavité (74, 274, 674), et
dans laquelle la partie de bande (108) de l'anneau (92, 292, 692) est configurée pour venir en prise avec une surface extérieure de la tige (80, 280, 680) pour empêcher la translation axiale de la tige (80, 280, 680) par rapport à la base (58, 258, 458, 658), et
dans laquelle l'ouverture (110, 710) est alignée avec la cavité (74, 274, 674) lorsque la partie de bande (108) vient en prise avec la surface extérieure de la tige (80, 280, 680) pour permettre à la tige (80, 280, 680) d'être déplacée par rapport à la tête (44, 244, 644) entre une première orientation et une deuxième orientation de telle sorte qu'au moins une partie de la tige (80, 280, 680) soit disposée dans la cavité (74, 274, 674).

2. Une fixation osseuse (242) selon la revendication 1, dans laquelle la tête (244) comprend une paroi définissant une cavité circonférentielle intérieure (254) et la paroi de la base (258) définit une cavité circonférentielle extérieure (264), les cavités circonférentielles étant alignées et configurées pour disposer un fil (260) selon une configuration pour fixer la tête (244) avec la base (258).

3. Une fixation osseuse (242) selon la revendication 2, dans laquelle le fil (260) comprend un fil de nitinol.

4. Une fixation osseuse (42) selon la revendication 1, dans laquelle la tête (44) comprend une paroi présentant une surface intérieure filetée (60) et la paroi de la base (58) comprend une surface extérieure filetée (64) pouvant venir en prise pour fixer la tête (44) avec la base (58).

5. Une fixation osseuse (42) selon la revendication 4, dans laquelle l'engrenage des surfaces filetées est minuté de telle sorte que l'agencement de la tête (44) et de la base (58) dans une orientation fixe aligne la cavité d'implant (50) dans une direction céphalique et/ou une direction caudale.

6. Une fixation osseuse (42, 242) selon la revendication 1, dans laquelle la première surface d'accouplement comprend au moins une surface plate et la deuxième surface d'accouplement (102, 302) comprend au moins une surface plate, les surfaces plates étant alignées pour fixer la base (58, 258) avec la couronne (88, 288).

7. Une fixation osseuse (42) selon la revendication 1, dans laquelle la première surface d'accouplement présente une configuration hexagonale et la deuxième surface d'accouplement (102) comprend une configuration hexagonale, les configurations hexagonales étant alignées pour fixer la base (58) avec la couronne (88).

8. Une fixation osseuse (42) selon la revendication 1, dans laquelle l'anneau (92) comprend une configuration annulaire elliptique.

9. Une fixation osseuse (42) selon la revendication 1, dans laquelle l'anneau (92) comprend une configuration en forme de U.

10. Une fixation osseuse (42, 242, 642) selon la revendication 1, dans laquelle, dans la première orientation, la tige (80, 280, 680) est coaxiale à l'axe (F, F1, F3), et dans la deuxième orientation, la tige (80, 280, 680) est disposée dans une plage de plus de 0 à 45 degrés relativement à l'axe (F, F1, F3).

11. Une fixation osseuse (42, 242, 642) selon la revendication 1, dans laquelle la tête (44, 244, 644) est rotative dans une orientation caudale dans une plage de 0-360 degrés.

12. Une fixation osseuse (42, 242, 642) selon la revendication 1, dans laquelle la tige (80, 280, 680) peut être disposée à l'intérieur de la cavité (74, 274, 674) selon une trajectoire S2-alar-iliaque.

13. Une fixation osseuse (42, 642) selon la revendication 1, dans laquelle la tête (44, 644) comprend une première surface d'appui et la base (58, 658) comprend une deuxième surface d'appui, les surfaces d'appui pouvant se mettre en prise pour fixer la tête (44, 644) avec la base (58, 658).

14. Une fixation osseuse (42, 242) selon la revendication 1, dans laquelle la couronne (88, 288) comprend une surface intérieure (94, 294) définissant une connexion de clé (104, 304) pouvant se mettre en prise pour faciliter la rotation de la couronne (88, 288) relativement à la base (58, 258).
